Europäisches Patentamt

European Patent Office

Office européen des brevets

(19)

(11) Publication number: **0 035 791**
**B1**

(12)

# EUROPEAN PATENT SPECIFICATION

(45) Date of publication of patent specification: **13.06.84**

(21) Application number: **81101762.3**

(22) Date of filing: **10.03.81**

(51) Int. Cl.³: **C 07 C 17/38,**
**C 10 H 23/00, C 07 C 17/34**
**//C07C21/06**

(54) A process for removing acetylene from reaction products of 1,2-dichloroethane pyrolysis.

(30) Priority: **12.03.80 DE 3009520**

(43) Date of publication of application:
**16.09.81 Bulletin 81/37**

(45) Publication of the grant of the patent:
**.13.06.84 Bulletin 84/24**

(84) Designated Contracting States:
**AT BE CH FR GB LI SE**

(56) References cited:
**DE - A - 2 438 153**
**US - A - 3 466 338**

(73) Proprietor: **WACKER-CHEMIE GMBH**
**Prinzregentenstrasse 22**
**D-8000 München 22 (DE)**

(72) Inventor: **Schmidhammer, Ludwig, Dr.**
**Pappelweg 5**
**D-8261 Haiming/Marktl (DE)**
Inventor: **Strasser, Rudolf, Dr.**
**Lindacher Strasse 58**
**D-8263 Burghausen (DE)**

(74) Representative: **Kraus, Walter, Dr. et al,**
**Patentanwälte Kraus & Weisert Irmgardstrasse**
**15**
**D-8000 München 71 (DE)**

Courier Press, Leamington Spa, England.

# Description

The invention relates to a process for removing acetylene by hydrochlorination from the reaction products obtained by thermal cracking or pyrolysis of 1,2-dichloroethane.

Modern large scale production of vinyl chloride from ethylene involves a multistage cyclic process comprising, among others, the following steps: chlorination of ethylene to form 1,2-dichloroethane; pyrolysis of 1,2-dichloroethane to produce vinyl chloride; and use of the hydrogen chloride obtained as pyrolysis product in an oxychlorination reaction with ethylene to again produce 1,2-dichloroethane.

During pyrolysis of 1,2-dichloroethane, minor amounts of acetylene are formed in addition to vinyl chloride and hydrogen chloride. Conventionally, the products of 1,2-dichloroethane pyrolysis are further processed by separating the hydrogen chloride from vinyl chloride and unreacted 1,2-dichloroethane whereupon the hydrogen chloride is recycled for use in the subsequent oxychlorination step. The acetylene is carried over with the hydrogen chloride and must be removed therefrom as, under oxychlorination conditions, acetylene forms a number of undesirable by-products in 1,2-dichloroethane which not only interfere with the pyrolysis reaction producing vinyl chloride but are detrimental to the polymerization of vinyl chloride as well.

To overcome the above problems, various methods of removing acetylene have been proposed:

German Auslegeschrift 15 68 679 recommends hydrogenation of the acetylene contained in hydrogen chloride with an excess of hydrogen in the presence of platinum or palladium catalysts. According to German Offenlegungsschrift 23 53 437, the efficiency of such a hydrogenation process will be improved by a catalyst bed wherein the profile of catalyst activity ascends in the direction of the flow of products.

However, hydrogenation in accordance with the above methods leads to a reduction in carbon yields since any process designed for complete conversion will produce useful ethylene together with useless ethane which is lost as an inert gas. Moreover, relatively large amounts of catalysts are required for maintaining the necessary rates of gas flow which are indispensable for a satisfactory space/time yield.

DE—A—2 438 153 discloses an improved support for use in such catalysts.

In other processes, acetylene is removed by hydrochlorination. As may be taken from U.S. patents 2,412,308 and 2,474,206, German patent 887 042, and British patent 731,844, such processes combine pyrolysis of 1,2-dichloroethane to vinyl chloride with the hydrochlorination of acetylene under use of hydrogen chloride; the acetylene formed during pyrolysis is replenished with fresh acetylene from an external source and then converted to vinyl chloride. However, because of today's high costs of acetylene, proceeding in the above manner has become unattractive economically.

Also known are methods of producing acetylene-free vinyl chloride; here, acetylene is removed from the product of 1,2-dichloroethane pyrolysis by first condensing off unreacted 1,2-dichloroethane and subsequently passing the remaining mixture of vinyl chloride and hydrogen chloride over a catalyst comprising mercuric chloride. on activated carbon, followed by the step of washing out hydrogen chloride with water (compare Belgian patent 564,178). German patent 1 100 616 discloses a modification of the above process wherein, before condensation of unreacted 1,2-dichloroethane, the major portion of hydrogen chloride is condensed by injecting steam into the pyrolysis zone.

Under economical aspects, the above described processes are not satisfactory either as the hydrogen chloride produced does not remain available for use in further oxychlorination steps but is lost in the form of hydrochlorid acid which is practically without value.

Another well known fact is that all of the previously mentioned methods fail to provide an adequate solution of the problem which resides in the poor service life or durability of the catalysts employed for the removal of acetylene, especially under the high operating pressures required for an economical space/time performance of the equipment: at elevated pressures, hydrogenation catalysts are highly susceptible to sooting or the formation of carbon black deposits thereon. Soot or carbon black deposits not only reduce the efficiency of the catalysts but promote as well the decomposition of acetylene under formation of additional carbon black. Moreover, at low acetylene contents, the efficiency of conventional hydrochlorination catalysts diminishes noticeable with a relatively short period of time as the catalysts are extremely sensitive to catalyst poisons while their catalytic action is directed towards continuous use rather than towards complete conversion.

Thus, it is the object of the invention to provide a catalyst system for the removal of acetylene by hydrochlorination under production of vinyl chloride, said catalyst system showing enhanced durability or service life at operating pressures of 8 to 20 bar.

According to the invention, the above object is achieved with the aid of a process for removing acetylene from the product of 1,2-dichloroethane pyrolysis by hydrochlorinating the acetylene in which the pyrolysis product is separated into a liquid and a gaseous product stream, said process comprising the step of exposing the gaseous product stream, prior to hydrochlorination, to a catalyst comprising platinum, palladium, ruthenium, rhodium in form of the respective metals and/or oxides and/or chlorides at temperatures of 50°C to 200°C and pressures of 8 to 20 bar absolute.

Preferably, operation is conducted at temperatures of 100° to 150°C.

The catalyst system of the invention comprises a first upstream catalyst bed of platinum, palladium, ruthenium, rhodium in form of the respective metals and/or oxides and/or chlorides and, following thereafter in direction of the flow of products, a second downstream bed of conventional hydrochlorination catalyst.

The catalyst beds of the invention may be accommodated either in two separate reactors arranged in series or in a single reactor.

The hydrochlorination catalyst normally employed in the process of the invention comprises mercuric chloride on a support of activated carbon. Also suited, however, are other catalysts known to promote the hydrochlorination reaction of acetylene.

As a rule, the noble metals and/or noble metal compounds constituting the first catalyst are deposited on a carrier or support but unsupported catalysts may also be used.

Illustrative of inert carriers are alumina, silica, silicates, titania, kieselguhr, activated carbon, and the like. It should be noted that the carriers are not restricted to those having a large surface area.

Illustrative of the platin, palladium, ruthenium and rhodium compounds suited for the invention are: platinum (II) oxide, platinum (IV) oxide, hexachloroplatinic acid, platinum (II) chloride, palladium (II) oxide, ruthenium (IV) chloride, ruthenium (VIII) oxide, rhodium (II) oxide, rhodium (III) chloride, rhodium hexachlorodate (III) dodecahydrate and rhodium (IV) oxide.

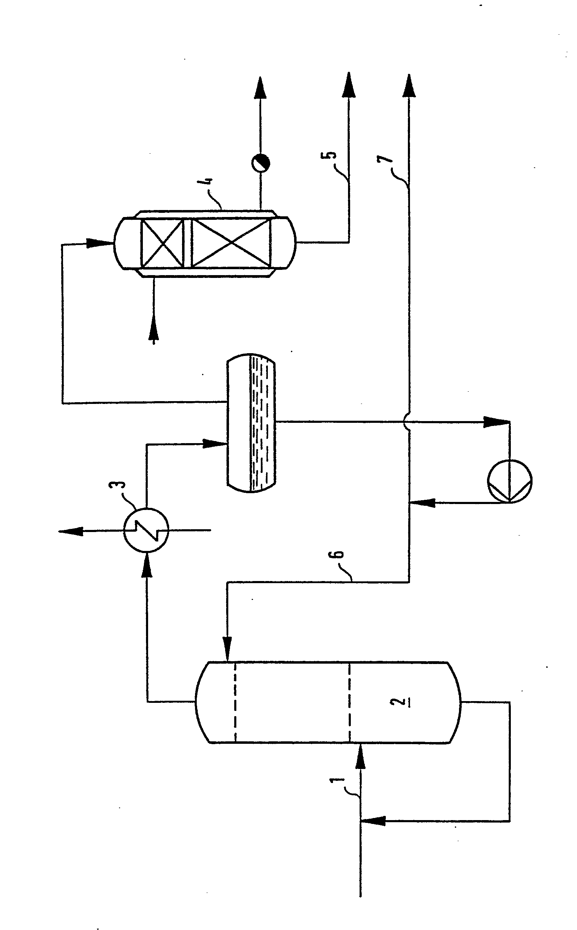

Below, the invention is illustrated under reference to the drawing which shows a block flow diagram of the process.

As shown in the drawing, the pre-cooled product of 1,2-dichloroethane pyrolysis, obtained as a vapor-liquid mixture, is fed via line 1 into quenching column 2 wherein the product is cooled down even further by vaporization while circulating continuously in the lower section of column 2. The vaporized product of pyrolysis is subsequently condensed in condenser 3 at a temperature of 30° to 50°C and a pressure of 8 to 20 bar. The non-condensable, gaseous product stream containing the major portion of the acetylene formed during pyrolysis is then passed over the catalyst bed of the invention. This zone containing noble metal and/or noble metal compounds is maintained at a temperature of from 50° to 200°C, preferably from 100° to 150°C, and a pressure of 8 to 20 bar absolute. Heating of the gaseous product stream to the respective temperature may be accomplished either by means of a heater arranged in front of the catalyst zone or by a heating jacket around the catalyst zone. The gaseous product stream is freed of acetylene and leaves the catalyst bed via line 5 to be fed into the hydrochlorination zone.

Part of the condensed liquid product stream is recycled as recycle stream 6 to quenching column 2 to effect some pre-purification of the reaction product while the remainder as liquid charge flows via line 7 into the hydrochlorination column from where pure hydrogen chloride substantially free of acetylene is withdrawn as an overhead, the bottoms comprising vinyl chloride as well as unreacted 1,2-dichloroethane.

The process of the invention permits removal of undesirable acetylen from the products of 1,2-dichloroethane pyrolysis. Surprisingly, it has been found that the service life of the hydrochlorination catalyst at pressures of 8 to 20 bar absolute may be enhanced considerably by placing the noble metal catalyst bed upstream of the bed of hydrochlorination catalyst. The carbon yield of the process is improved since acetylene is converted into the desired product, vinyl chloride, while, on the other hand, the formation of carbon black deposits which promote spontaneous decomposition of acetylene to more carbon black is suppressed. Moreover, properly purified, the hydrogen chloride obtained in the process of the invention may be used without detrimental effects for the oxychlorination of ethylene.

Example 1

After condensation at 35°C and a pressure of 9 bar, there were obtained 1500 Nl/hour of a gaseous reaction product having the following approximate composition:

75 mole % hydrogen chloride,
24.7 mole % vinyl chloride,
0.13 mole % 1,2-dichloroethane,
0.17 mole % acetylene,

together with 7.9 kg/hour of liquid reaction product containing not more than 0.0005 mole % of acetylene.

The gaseous reaction product was passed over a catalyst bed in a vertically arranged reactor (diameter 55 mm, length 210 cm). Seen from the inlet of the reactor, the catalyst bed was composed of one liter of noble metal catalyst (depth of catalyst layer 50 cm) comprising 0.15% by weight of palladium on kieselguhr, followed by three litres of hydrochlorination catalyst (depth of layer 155 cm) comprising 10% by weight of $HgCl_2$ on activated carbon (EKT 4 by Lurgi). The reactor had a steam jacket, and the temperature of the catalyst bed was maintained at about 100°C with the aid of condensable steam. Located at the outlet of the reactor was a pressure valve for maintaining the pressure in the catalyst bed at 10 bar absolute.

Even after two months of operation, the acetylene content of the product stream leaving the reactor remained in the order of less than 10 ppm by volume.

Comparative Example 1

The operating conditions were the same as in Example 1 with the exception that, instead of the noble metal catalyst of the invention, the upper zone of the reactor contained one liter of glass Raschig rings as inert material.

At the start of the experiment, the acetylene content of the product stream leaving the reactor was in the order of less than 10 ppm by volume. After an operating period of one month, the acetylene content had risen to 30 ppm by volume, and after two months, to 115 ppm by volume.

### Example 2

Condensation at 40°C and 9 bar resulted in 1000 Nl/hour of a gaseous reaction product having the following approximate composition:

73.5 mol % hydrogen chloride,
26.1 mole % vinyl chloride,
0.20 mole % 1,2-dichloroethane,
0.20 mole % acetylene,

together with 5.3 kg/hour of liquid reaction product containing only 0.0006 mole % of acetylene.

At 150°C and a pressure of 9 bar, the gaseous reaction product was passed through the reactor described in Example 1, the catalyst bed therein—seen from the inlet of the reactor—being composed of one liter of noble metal catalyst (depth of layer about 50 cm) comprising 0.2% by weight of ruthenium on granular titania, followed by three liters of hydrochlorination catalyst (depth of layer about 155 cm) comprising 15% by weight of $CeCl_3 . 4HgCl_2$ on activated carbon (EKT 4).

Even after two months of operation, the acetylene content of the product stream leaving the reactor remained in the order of 10 ppm by volume.

### Comparative Example 2

The operating conditions were the same as in Example 2, with the exception that the upper zone of the reactor was filled with one liter of glass Raschig rings as inert material to replace the noble metal catalyst of the invention.

At the start of the experiment, the acetylene content of the product stream at the outlet of the reactor was in the order of 10 ppm by volume. After two weeks of operation, the acetylene content had risen to 40 ppm by volume, reaching a height of 70 ppm by volume after one month, and of 130 ppm by volume after two months.

The maximum tolerable concentration of acetylene is considered to be about 50 ppm by volume. When working in accordance with the invention, the acetylene content actually observed after two months of operation falls below the above value by a factor of at least 5. In contrast thereto, hydrochlorination catalysts used without the upstream bed of noble metal catalyst are exhausted after only one month of operation.

## Claims

1. A process for removing acetylene from the product of thermal pyrolysis of 1,2-dichloroethane by hydrochlorinating acetylene in which the reaction product is separated into a liquid and a gaseous product stream, comprising the step of exposing said gaseous product stream, prior to hydrochlorination, to a catalyst comprising platinum, palladium, ruthenium, rhodium in form of the respective metals and/or oxides and/or chlorides at temperatures of 50° to 200°C and pressures of 8 to 20 bar absolute.

2. The process according to Claim 1 wherein said gaseous product stream is exposed to said catalyst comprising platinum, palladium, ruthenium, rhodium in form of the respective metals and/or oxides and/or chlorides at temperatures of 100° to 150°C.

## Patentansprüche

1. Verfahren zur Entfernung von Acetylen aus dem Produkt der thermischen Pyrolyse von 1,2-Dichlorethan durch Hydrochlorierung von Acetylen, wobei das Reaktionsprodukt in einen flüssigen und einen gasförmigen Produktstrom aufgeteilt wird, gekennzeichnet durch die Stufe, in der man den genannten gasförmigen Produktstrom vor der Hydrochlorierung einem Katalysator, enthaltend Platin, Palladium, Ruthenium, Rhodium in Form der jeweiligen Metalle und/oder Oxide und/oder Chloride, bei Temperaturen von 50 bis 200°C und Drücken von 8 bis 20 bar absolut aussetzt.

2. Verfahren nach Anspruch 1, dadurch gekennzeichnet, daß man den genannten gasförmigen Produktstrom dem · Katalysator, enthaltend Platin, Palladium, Ruthenium, Rhodium in Form der jeweiligen Metalle und/oder Oxide und/oder Chloride, bei Temperaturen von 100 bis 150°C aussetzt.

## Revendications

1. Procédé d'élimination de l'acétylène du produit de la pyrolyse thermique du 1,2-dichloroéthane par hydrochoruration de l'acétylène dans lequel le produit de la réaction est séparé en un courant de produit liquide et un courant de produit gazeux, caractérisé en ce qu'il comprend l'étape d'exposer ledit courant de produit gazeux, avant l'hydrochloruration, à un catalyseur comprenant du platine, palladium, ruthénium, rhodium sous la forme des métaux et/ou oxydes et/ou chlorures respectifs à des températures de 50 à 200°C et à des pressions de 8 à 20 bars absolus.

2. Procédé suivant la revendication 1, caractérisé en ce que ledit courant de produit gazeux est exposé audit catalyseur comprenant du platine, palladium, ruthénium, rhodium sous la forme des métaux et/ou oxydes et/ou chlorures respectifs à des températures de 100 à 150°C.